(19) Europäisches Patentamt
European Patent Office
Office européen des brevets

(11) EP 3 404 005 A1

(12) EUROPEAN PATENT APPLICATION

(43) Date of publication:
**21.11.2018 Bulletin 2018/47**

(51) Int Cl.:
***C07C 2/12** (2006.01)*

(21) Application number: **17305565.8**

(22) Date of filing: **17.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Total Research & Technology Feluy**
  **7181 Seneffe (BE)**
- **Centre National de la Recherche Scientifique (CNRS)**
  **75016 Paris (FR)**

(72) Inventors:
- **BORODINA, Elena**
  **1180 BRUXELLES (BE)**
- **MINOUX, Delphine**
  **1400 NIVELLES (BE)**
- **NESTERENKO, Nikolai**
  **1402 NIVELLES (BE)**
- **DATH, Jean-Pierre**
  **7970 BELOEIL (BE)**
- **ANDREI, Radu Dorin**
  **34090 MONTPELLIER (FR)**
- **HULEA, Vasile**
  **34090 MONTPELLIER (FR)**
- **CAMMARANO, Claudia**
  **34000 MONTPELLIER (FR)**

(74) Representative: **Mazurelle, Jean et al**
**Total Research and Technology Feluy**
**Zone Industrielle Feluy C**
**7181 Seneffe (BE)**

# (54) PROCESS FOR ETHYLENE OLIGOMERISATION FROM DILUTED STREAM

(57) The invention relates to a process for the conversion of ethylene into olefins having at least four carbon atoms wherein the process comprises:
a) providing feedstream comprising from 0.5 to 50 wt% of ethylene as based on total weight of the feedstream;
b) putting the feedstream in contact with a catalyst at a temperature ranging from 80 to 500°C, under a pressure ranging from 0.5 to 2.9 MPa; and at a weight hourly space velocity (WHSV) of the feedstream ranging from 0.1 to 10.0 $h^{-1}$;
c) recovering the olefins having at least four carbon atoms;
and further wherein the catalyst of step b) comprises a mesoporous material with one or more metals of group VIIIB and/or of group VIB as active phase

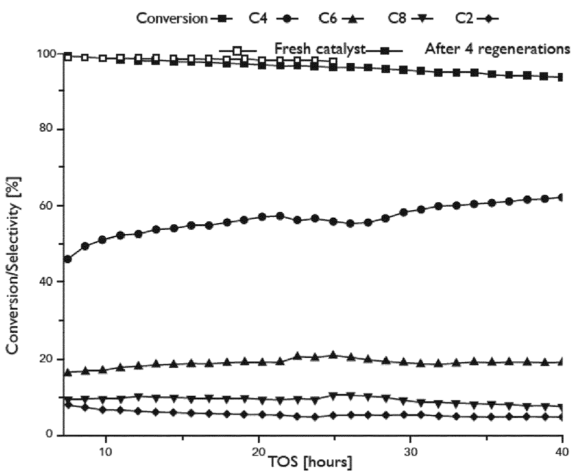


Figure 1

EP 3 404 005 A1

## Description

### Field of the invention

The invention relates to a process of ethylene valorization from diluted streams of varied origin at low pressure in order to reduce a waste of ethylene containing gas. This ethylene is directed to the production of more valuable liquids.

### Background of the invention

Refinery off-gases, e.g., Fluid Catalytic Cracking (FCC) off-gas and Delayed Cocker Unit (DCU) off-gas, contain significant amount of ethylene (5-50 wt% based on the total weight of the stream) and nowadays are burned as fuel gas. From both economical and environmental point of view, it could be interesting to recover this wasted ethylene. Moreover, development of Oxidative Coupling of Methane (OCM) technology gives even more ethylene on the market, which needs to be valorized into more easy transportable liquid products. The important sources are not in the competition with the ethylene consumption market like polyethylene for example. Therefore from these ethylene streams others types of products can be considered to be formed, such as oligomers with carbon atoms higher than four (C4+).

Few industrial technologies including heterogeneous catalysis of ethylene transformation from diluted stream into liquids exist. The end products of all these inventions are hydrocarbons of at least five carbon atoms (C5+), liquid, applied as motor fuels. Most of the technologies are using metal exchanged (typically Zn or Ga) ZSM-5 as a catalyst and low pressure (0.1 to 1 MPa). The product composition can account for up to 30 wt% of Liquefied Petroleum Gas (LPG) as based on the total weight of the production composition, reducing the carbon efficiency of the process. Higher carbon efficiency (less than 9 wt% of LPG) can be achieved with the use of Ni-$SiO_2$/$Al_2O_3$ catalyst, but the process requires high pressure which severely increases the costs.

In "Heterogeneous oligomerization of ethylene over highly active and stable Ni-Al-SBA-15 mesoporous catalysts" by Andrei, R. D. et al, Journal of Catalysis, 323 (2015) 76-84; Ni-Al-SBA-15 catalyst has been shown to have superior performance in terms of activity and stability compared to known catalysts (Ni-zeolites, Ni-$SiO_2$/$Al_2O_3$, Ni-MCM-41) for oligomerization of concentrated ethylene (at least 99.9 wt% of ethylene based on the total weight of the feedstream) at high pressure (≥ 29 barg).

In "Nickel and molybdenum containing mesoporous catalysts for ethylene oligomerization and metathesis"by Andrei, R. D. et al, New J. Chem 40 (2016) 4146-4152; Ni-Al-$SiO_2$ (where $SiO_2$ is a non-ordered mesoporous silica) catalyst was evaluated for oligomerization of concentrated ethylene (at least 99.9 wt%) at high pressure 3.0 MPa and showed a conversion of 80% of ethylene with a selectivity of about 50 % to C4.

Thus there is still a need for a process to convert the refinery off-gases, such as fluid catalytic cracking (FCC) off-gases, into valuable products without the need of purification of the stream to concentrate it. There is a need for a process to convert refinery off-gases comprising ethylene to olefin having at least four carbon atoms with high carbon efficiency, high selectivity toward olefin having four carbon atoms and which is cost efficient.

It is an object of the invention to provide a new process and new use of a catalyst for the conversion of a diluted stream of ethylene into olefin having at least four carbon atoms that is cost effective and has high carbon efficiency. It is also an objective of the invention to provide a new process and new use of a catalyst that further shows an improved selectivity toward olefin having four carbon atoms.

### Summary of the invention

According to a first aspect, the invention provides a process for the conversion of ethylene into olefins having at least four carbon atoms wherein the process comprises:

a) providing a feedstream comprising from 0.5 to 50 wt% of ethylene as based on the total weight of the feedstream;
b) putting the feedstream in contact with a catalyst at a temperature ranging from 80 to 500°C, under a pressure ranging from 0.5 to 2.9 MPa; and at a weight hourly space velocity (WHSV) of the feedstream ranging from 0.1 to 10.0 $h^{-1}$;
c) recovering the olefins having at least four carbon atoms;

further wherein the catalyst of step b) consists of one or more metals of group VIIIB and/or of group VIB deposited on a support being a mesoporous material.

The WHSV is determined on the total feed.

It has been found by the inventors that, surprisingly, catalysts consisting of consists of one or more metals of group VIIIB and/or of group VIB (according to the CAS classification) deposited on a support being a mesoporous material can be used in an oligomerization reaction to convert ethylene from diluted stream at low reaction pressure with high

carbon efficiency of more than 80% and with a high selectivity to olefin having at least four carbon atoms (C4+) of more than 95 %. The invention permits using FCC off gases as fuel gases in order to upgrade them into valuable products without the need of high cost compression and without the need of any purification of the stream. In addition, it has been found by the inventors that conducting the reaction at low WHSV of 10.0 $h^{-1}$ or less combined with a diluted stream of ethylene increases the contribution of olefins having four carbon atoms in the product distribution, which is of great interest for use as feedstock in propylene production by Olefin Cracking Process (OCP). Indeed, as shown in the examples the selectivity toward C4 can be of 60 wt% or more as based on the total weight of the products.

With preference, one or more of the following features can be used to better define the catalyst of step b):

- The mesoporous material is an ordered mesoporous material or any type of mesoporous material based on silica. The mesoporous material is an Al-containing material, preferably Al modified silica type.
- The mesoporous material is a mesopourous material of SBA-15 topology or is a mesopourous material of Al-modified silica type.
- The one or more metals of group VIIIB and/or of group VIB of the catalyst of step b) are selected from nickel, cobalt, chromium, molybdenum, tungsten, palladium and any mixture thereof.
- The one or more metals of group VIIIB and/or of group VIB is a mixture of nickel with one or more metals selected from cobalt, chromium, molybdenum, tungsten and any mixture thereof, preferably wherein the mixture comprises more than 50 wt% of nickel as based on the total weight of the mixture, more preferably more than 70 wt% of nickel.
- The catalyst of step b) consists of nickel deposited on a support being a mesoporous material, preferably on a support being an aluminated ordered mesoporous material.
- The catalyst of step b) consists of nickel deposited on a support being a mesoporous material, and the catalyst comprises from 0.5 to 10.0 wt% of nickel based on the total weight of the catalyst as determined according to UOP961-12, preferably from 1.0 to 5.0 wt%, more preferably from 2.0 to 3.0 wt%.
- The catalyst of step b) consists of nickel deposited on a support being a mesoporous material of SBA-15 topology, preferably the catalyst is Ni-Al-SBA-15.
- The catalyst of step b) consists of nickel deposited on a support being a mesopourous material of Al-modified silica type, preferably the catalyst is Ni-Al-$SiO_2$.
- The mesoporous material of the catalyst comprises mesoporous pores having an average diameter ranging from 2 to 50 nm, as determined according to ASTM D 4641
- 94 (reapproved 2006), preferably ranging from 5 to 40 nm.
- The mesoporous material of the catalyst comprises mesoporous pores having an average diameter of at least 2 nm, preferably of at least 5 nm, more preferably of at least 7 nm as determined according to ASTM D 4641 - 94 (reapproved 2006), and even more preferably of at least 7.5 nm.
- The mesoporous material of the catalyst comprises mesoporous pores having an average diameter of at least 2 nm as determined according to ASTM D 4641 - 94 (reapproved 2006) and a mesoporous pore volume of at least 0.1 mL/g as determined according to ASTM D 4641 - 94 (reapproved 2006).
- The catalyst comprises an ordered mesoporous silica material or any other silica exhibiting a type IV isotherm.
- The catalyst has a total surface area ranging from 100 $m^2$/g to 1000 $m^2$/g, preferably from 120 to 700 $m^2$/g, more preferably from 150 $m^2$/g to about 500 $m^2$/g as determined by $N_2$ sorption analysis according to ASTM D 4365 - 95 (reapproved 2008).
- The catalyst has a bulk Si/Al ratio ranging from 5 to 10 preferably from 6 to 8 as determined according to UOP961-12.
- The catalyst has a sieve fraction ranging from 0.10 to 0.50 mm, preferably from 0.15 to 0.25 mm as measured according to ASTM D4513 - 11.

With preference, one or more of the following features can be used to further define the inventive process:

- The feedstream is put into contact with the catalyst at a temperature ranging from 100°C to 400°C, preferably from 150 to 350°C, more preferably at a temperature ranging from 180 to 280°C.
- The feedstream is put into contact with the catalyst at a pressure ranging from 0.7 to 2.0 MPa, and most preferably from 1.0 to 1.5 MPa.
- The feedstream is put into contact with the catalyst at an ethylene partial pressure of at most 1.45 MPa, preferably of at most 1.0 MPa, more preferably of at most 0.8 MPa, and even more preferably of at most 0.5 MPa and even more preferably of at most 0.4 MPa.
- The reaction of step b) is performed at a weight hourly space velocity (WHSV) of the feedstream of from 0.5 to 8.0 $h^{-1}$, preferably from 1.0 to 5.0 $h^{-1}$.
- The feedstream comprises at least 2 wt% of ethylene based on the total weight of the feedstream, preferably at least 3 wt% of ethylene, more preferably at least 5 wt% ethylene, even more preferably at least 10 wt% of ethylene, most preferably at least 15 wt% of ethylene.

- The feedstream comprises at most 45 wt% of ethylene based on the total weight of the feedstream, more preferably at most 40 wt%, even more preferably at most 35 wt% and most preferably at most 30 wt%.
- The feedstream comprises at most 10000 wt ppm of water based on the total weight of the feedstream, preferably at most 1000 wt ppm, more preferably at most 500 wt ppm.
- The process is carried out without replacement or reactivation of the catalyst during more than 40 hours, preferably more than 50 hours, more preferably more than 60 hours, and even more preferably more than 70 hours.
- The feedstream comprises from 0.1 to 5.0 wt% of CO as based on the total weight of the feedstream, preferably less than 2.0 wt%, more preferably less than 1.0 wt%.
- The feedstream comprises from 2.0 to 20.0 wt% of hydrogen as based on the total weight of the feedstream, preferably less than 4.0 wt%
- The feedstream comprises less than 90 wt ppm of CO and less than 1.0 wt% of hydrogen as based on the total weight of the feedstream.
- The feedstream comprises from 0.1 to 5.0 wt% of $CO_2$ as based on the total weight of the feedstream, preferably less than 3.0 wt%, more preferably less than 1.0 wt%.
- The feedstream comprises less than 0.01 wt% of $H_2S$ and $NH_3$ as based on the total weight of the feedstream.
- The olefins recovered at step c) comprise at least 50 wt% of olefins having four carbon atoms based on the total weight of the recovered olefins.
- The olefins recovered at step c) comprise less than 10 wt% of olefins having 3 carbon atoms based on the total weight of the recovered olefins.

According to a second aspect the invention provides the use of a catalyst consisting of one or more metals of group VIIIB and/or of group VIB deposited on a support being a mesoporous material, in a process to convert ethylene into olefins having at least four carbon atoms according to the above definition wherein the process comprises a step wherein the feedstream is put in contact with said catalyst at a temperature ranging from 80 to 500°C, under a pressure ranging from 0.5 to 2.9 MPa and at a weight hourly space velocity (WHSV) of the feedstream ranging from 0.1 to 10.0 $h^{-1}$; and in that the feedstream comprises from 0.5 to 50 wt% of ethylene as based on total weight of the feedstream.

Preferably, the one or more metals of group VIIIB and/or of group VIB of the catalyst are selected from nickel, cobalt, chromium, molybdenum, tungsten, palladium and any mixture thereof, more preferably nickel or cobalt.

With preference, the catalyst is nickel deposited on a mesoporous material of SBA-15 topology or nickel deposited on a mesoporous material of Al-modified silica type.

With preference, the feedstream comprising ethylene is an effluent of a process selected from Methanol To Olefin process (MTO), ethane/naphtha cracker process or oxidative coupling of methane process (OCM), or is a fluid catalytic cracking (FCC) off-gases used to produce gasoline, diesel and/or jet additives.

**Description of the figures:**

- Figure 1 illustrates, for Ni-Al-SBA-15 catalysts, the conversion and selectivity of ethylene oligomerization from diluted streams at P = 1.5 MPa, T = 250 °C, 17 wt% C2- in $N_2$ stream as based on the total weight of the stream, WHSV (feed) = 0.75 $h^{-1}$.
- Figure 2 illustrates, for Ni-Al-SBA-15 catalyst, the influence of temperature on conversion and selectivity of ethylene oligomerization from diluted streams at reaction temperatures 200 and 250 °C after 40 hours TOS, at P = 1.5 MPa, 17 wt% C2- in $N_2$ stream as based on the total weight of the stream, WHSV (feed) = 0.75 $h^{-1}$.
- Figure 3 illustrates, for Ni-Ai-SBA-15 catalyst, the influence of temperature on conversion and selectivity of ethylene oligomerization from diluted streams at reaction temperatures 200 and 250 °C after 10 hours TOS at P = 1.5 MPa, 17 wt% C2- in $N_2$ stream as based on the total weight of the stream, WHSV (feed) = 3.5 $h^{-1}$.
- Figure 4 illustrates, for Ni-Ai-SBA-15 catalyst, the influence of WHSV on conversion and selectivity of ethylene oligomerization from diluted streams at WHSV (feed) of 1.75 and 3.5 $h^{-1}$ after 10 hours TOS at T = 250 °C, P = 1.5 MPa, 17 wt% C2- in $N_2$ stream as based on the total weight of the stream.
- Figure 5 illustrates, for Ni-Ai-SBA-15 catalyst, the space time yield of ethylene oligomerization from diluted streams at WHSV (feed) 0.75, 1.75 and 3.5 $h^{-1}$ after 10 hours TOS at T = 250 °C, P = 1.5 MPa, 17 wt% C2- in $N_2$ stream as based on the total weight of the stream.
- Figure 6 illustrates, for Ni-Al-$SiO_2$ catalyst, the selectivity of ethylene oligomerization from diluted streams at WHSV (feed) of 1.1 $h^{-1}$ at T = 250 C, P = 1.5 MPa, 17.0 wt% C2- in $N_2$ stream as based on the total weight of the stream.

## Detailed description of the invention

As used herein the term "catalyst" refers to a "supported catalyst" which is a catalyst comprising an active phase and a support. According to the invention, the active phase consists in one or more metals of group VIIIB and/or of group VIB, preferably nickel; and the support is a mesoporous material.

The invention relates to the conversion of ethylene from a diluted feedstream having at most 50 wt% of ethylene, into olefins having at least four carbon atoms via an oligomerization reaction in presence of a specific catalyst having one or more metals of group VIIIB and/or of group VIB as active phase, preferably the active phase is or contains nickel, and a support being a mesoporous material, said reaction being conducted at low pressure and at low weight hourly space velocity.

The invention relates to a process for the conversion of ethylene into olefins having at least four carbon atoms wherein the process comprises:

a) providing a feedstream comprising from 0.5 to 50 wt% of ethylene as based on the total weight of the feedstream;
b) putting the feedstream in contact with a catalyst at a temperature ranging from 80 to 500°C, under a pressure ranging from 0.1 to 2.9 MPa, and at a weight hourly space velocity (WHSV) of the feedstream ranging from 0.1 to 10.0 $h^{-1}$;
c) recovering the olefins having at least four carbon atoms ;

further wherein the catalyst of step b) consists of one or more metals of group VIIIB and/or of group VIB deposited on a support being a mesoporous material.

The invention provides a process to convert a diluted stream of ethylene into olefins having at least four carbon atoms, wherein the diluted feedstream comprises at least 5 wt% of ethylene as based on the total weight of the feedstream, preferably at least 10 wt%, more preferably at least 15 wt%. In an embodiment of the invention, the feedstream comprises at most 50 wt% of ethylene as based on the total weight of the feedstream, preferably at most 45 wt%, more preferably at most 40 wt%, even more preferably at most 35 wt% and most preferably at most 30 wt%.

According to the invention, the feedstream comprises ethylene diluted into inert component such as a mixture of $CH_4$, $C_2H_6$, $C_3H_8$, $N_2$. The feedstream can have a typical composition of an FCC off gases i.e. the feedstream can comprise ethylene diluted into a mixture of $CH_4$, $C_2H_6$, $C_3H_8$, $N_2$ together with C3 and C4 olefins.

However, the feedstream may also comprise some contaminants such as carbon oxide (CO), carbon dioxide ($CO_2$), hydrogen ($H_2$) and $H_2O$. In such a case, the feedstream preferably comprises from 0.1 to 5.0 wt% of CO, preferably less than 2.0 wt% more preferably less than 1.0 wt% CO. Preferably, the feedstream comprises from 0.1 to 5.0 wt% of $CO_2$, preferably less than 3.0 wt% $CO_2$. Preferably, the feedstream comprises from 2.0 to 20.0 wt% of hydrogen, preferably less than 4.0 wt% $H_2$. All wt% are based on the total weight of the feedstream. Preferably the feedstream comprises less than 1.0 wt% of $H_2$ based on the total weight of the feedstream.

In an embodiment, the feedstream comprises less than 0.01 wt% of $H_2S$ and $NH_3$ as based on the total weight of the feedstream.

In an embodiment, the feedstream comprises at most 10000 wt ppm of water based on the total weight of the feedstream, preferably at most 1000 wt ppm, more preferably at most 500 wt ppm. When the feedstream comprises more than 10000 wt ppm it is preferable to dry the feedstream.

The process is carried out in an installation comprising:

- an optional purification section consisting of amine absorber, water wash unit, guard bed and drying section;
- a batch (CSTR), fixed or fluidized bed oligomerisation reactor, preferably in a fixed catalyst bed mode preferably equipped with an heat exchange system;
- lines to introduce the feedstream to the reactor and remove products from the reactor;
- an oligomerisation separation device to separate gas and liquid products;
- a temperature sensor and controller for detecting and controlling the temperature of the reactor at a reaction temperature ranging from 80 to 500°C;
- flow controllers to control the weight hourly space velocity (WHSV) of the feedstream to the reactor to a WHSV of from 0.1 to 10.0 $h^{-1}$; and
- a pressure controller to control the reactor pressure to a pressure ranging from 0.1 to 2.9 MPa.

The installation according to the invention can further comprise a separation device to recover unreacted ethylene and lines to recycle said unreacted ethylene into the reactor.

In a preferred embodiment, the process is carried out at a reaction temperature of at least 100°C, preferably of at least 150°C, more preferably of at least 180°C and even more preferably of at least 200°C. In an embodiment, the temperature is kept below 500°C, preferably below 450°C, more preferably below 400°C and even more preferably

below 350°C. The preferred range for the reaction temperature is between 200 to 300°C. The person skilled in the art may increase the temperature to raise the conversion rate to olefins having 4 atoms of carbon or more. However a temperature of about 200°C is preferred regarding the stability of the catalyst.

In another embodiment, the pressure is at least 0.5 MPa, preferably at least 0.7 MPa, more preferably at least 1.0 MPa, even more preferably at least 1.2 MPa and even more preferably at least 1.4 MPa. In another embodiment, the pressure is at most 3.0 MPa, preferably at most 2.8 MPa, more preferably at most 2.5 MPa and even more preferably at most 2.0 MPa.

In a preferred embodiment of the invention the process is carried out at an ethylene partial pressure of at most 1.0 MPa, preferably of at most 0.8 MPa, more preferably of at most 0.5 MPa and even more preferably of at most 0.4 MPa.

In a mixture of gases, each gas has a partial pressure which is the hypothetical pressure of that gas if it alone occupied the entire volume of the original mixture at the same temperature. The total pressure of an ideal gas mixture is the sum of the partial pressures of each individual gas in the mixture.

It relies on the following isotherm relation:

$$\frac{px}{ptot} = \frac{nx}{ntot}$$

- $p_x$ is the partial pressure of gas X
- $p_{tot}$ is the total pressure of the gas mixture
- $n_x$ is the amount of substance of gas (X)
- $n_{tot}$ is the total amount of substance in gas mixture

The weight hourly space velocity (WHSV) is the quotient of the mass flow rate of the reactants divided by the mass of the catalyst in the reactor. In a preferred embodiment, the reaction is performed at a WHSV of at most 10.0 $h^{-1}$, preferably ranging from 0.2 to 9.0 $h^{-1}$, more preferably ranging from 0.5 to 8.0 $h^{-1}$, and even more preferably ranging from 1.0 to 5.0 $h^{-1}$. The low WHSV combined with the dilution of ethylene and the use of the catalyst according to the invention has been shown to have an effect on the product distribution of C4 and C6. Indeed, it has been shown that low WHSV increases the contribution of C4 over C6 (see examples).

In a preferred embodiment the process can be carried out with a stable performance with respect to activity and selectivity during more than 40 hours, preferably more than 50 hours, more preferably more than 60 hours, and even more preferably more than 70 hours without the need of reactivation or replacement of the supported catalyst.

In an embodiment, the process is carried out in a fixed bed or in a fluidized bed reactor comprising at least one catalytic bed. Such reactors are well-known from the person skilled in the art and for instance described in EP2257366 or in US7279138

If the feed contains higher content of impurities, purification section can be added optionally to remove CO, $CO_2$, $H_2$ or $H_2O$. Optionally, the following units can be introduced: amine absorber to remove $H_2S$, water wash unit to remove amine from amine adsorber and decrease content of $NH_3$ and $CO_2$. Optionally, a guard bed can be implemented to remove impurities such as CO, $CO_2$, $H_2S$ and/or $NH_3$. Optionally, a drying section can be added to remove $H_2O$, which can have a negative effect on catalyst performance.

<u>Catalyst</u>

The present invention contemplates the use of a catalyst being a supported catalyst in oligomerization process to convert ethylene into olefins having at least four carbon atoms (C4+) wherein ethylene is diluted in the feedstream. The catalyst used in the invention is a calcined supported catalyst thus any reference to the catalyst includes a reference to a calcined supported catalyst.

The catalyst consists of one or more metals of group VIIIB and/or of group VIB deposited on a support being a mesoporous material. The one or more metals of group VIIIB and/or of group VIB are preferably selected from nickel, cobalt, chromium, molybdenum, tungsten, palladium and/or any mixture thereof. In a preferred embodiment, the active phase is a mixture of nickel with one or more metals of group VIIIB and/or of group VIB selected from cobalt, chromium, tungsten and molybdenum, preferably wherein the mixture comprises more than 50 wt% of nickel as based on the total weight of the mixture, more preferably more than 70 wt% of nickel. In another preferred embodiment nickel is the active phase of the catalyst.

According to an embodiment, the mesoporous material is obtained from a zeolitic material of the FAU, MFI, MOR or FER type framework by various post-treatment procedures. The mesoporized zeolites possesses a residual network of micropores (ie pores < 2 nm in diameter) and contains mesopores (pores with diameter in the range 2-50 nm) connected to the micropores, and a ratio of the volume of the mesopores to the volume of the micropores in the

range 0.2 to 3. Further the said materials may be shaped with a binder. The preferred zeolite is FAU (zeolite Y).

The mesoporization may include steaming followed by a leaching procedure to remove the extra framework aluminum.

The mesoporisation of zeolite porosity may include the following steps:

- suspending a zeolite or a composite material comprising it in a basic aqueous solution comprising at least one strong base, e.g NaOH or KOH, and/or a weak base, in particular, sodium carbonate, sodium citrate, ammonium hydroxide etc., for example, at a concentration ranging from 0.001 to 2 M, at room or elevated temperature (25-200°C), optionally an organic base may be present together with inorganic,
- neutralizing the medium by addition of at least one acid solution with pH<5, for example, at a concentration ranging from 0.005 to 2 M, at room temperature,
- separating the zeolite obtained from the solution and washing it with a solvent, especially a polar solvent, for example, water,
  optionally drying the washed zeolite,
- placing the washed and optionally dried zeolite in contact with a solution to exchange back the alkaline cations, especially an aqueous solution, of NH4NO3, especially at a concentration ranging from 0.01 to 0.5 M; this step can be performed several times, for example 2 to 3 times,
- washing the zeolite with water,
- calcining the zeolite obtained, and recovering the zeolite.

Preferably, the mesoporous material is an ordered mesoporous material or any type of mesoporous material based on silica. Indeed, among the solids which can be used, the mesoporous silicas or silica-alumina have a large specific surface area (preferably above 400 $m^2/g$, more preferably above 600 $m^2/g$) and a mesoporous structure with pores of uniform size which would overcome the constraints related to the diffusion of coarse particles molecules.

The mesoporous silicas with ordered structures are obtained by synthesis from a silicic precursor in the presence of structuring agents which are micelles of surface-active agents. An amorphous silica is obtained having a porous structure ordered on the scale of a few nanometers. There are currently a number of structured mesoporous silicas developed by the different surfactant crosslinks / silica precursors. Among the mesostructured porous materials, it can be distinguished :

- the mesoporous silicas of the M41 S family, which comprise MCM-41 type materials with hexagonal 2D crystallographic structure (p6mm group), MCM-48 type materials having a cubic structure (Ia3d) and MCM-50 materials having a lamellar structure;
- the mesoporous silicas of type SBA (Santa Barbara Amorphous). Among this type of materials are: SBA-1 (cubic), SBA-15 (hexagonal) SBA-16 (cubic), SBA-14 (lamellar) SBA-12 (hexagonal);
- the mesoporous silicas of the MCF type (Mesostructured Cellular Foam), which are obtained by adding swelling agents such as TMB (1,3,5-trimethylbenzene) to the synthesis of SBA-15, which leads to enlargement of the micelles. This makes it possible to obtain a structure consisting of uniform large pores. These materials have high thermal stability.
- the MSU-type mesoporous silicas (Michigan State University), these materials are obtained from non-ionic surfactants or from tri-block copolymers.

Since the mesoporous silica matrices are not acidic, it is necessary to provide them with acidity and a metallic function (Ni) for use in ethylene's oligomerization. The acidity can be provided either by inserting aluminum dispersed in the silica network by direct synthesis [C.T. Kresge, M. E. Leonowicz, W.J. Roth, J.C. Vartuli, J.S. Beck, Nature 1992, 359, 710; A. Corma, V. Fornes, T. Navarro, J. Perez-Pariente, Journal of Catalysis 1994, 148, 569], or by post-synthesis grafts (alumination) with reagents such as $AlCl_3$ [ Mokaya, Journal of Catalysis 2000, 193, 103], $Al(NO_3)_3$ [S. C. Shen, S. Kawi, Chemistry Letters 1999, 28, 1293], Al $(O\text{-}i\text{-}Pr)_3$ [R. Mokaya, W. Jones, Chemical Communications 1997, 2185], sodium aluminate [Z. Luan, M. Hartmann, D. Zhao, W. Zhou, L. Kevan, Chemistry of materials, 1999, 11, 1621]. The presence of aluminum confers on the solids ion exchange sites as well as acidic sites of Brønsted type and Lewis type. Several studies have addressed the characterization of acidity by TPD of $NH_3$ or by adsorption of pyridine followed by infrared (FTIR) [A. Jentys, N.H. Pham, H. Vinek, Journal of the Chemical Society, Faraday Transaction 1996, 62, 3287]. These studies demonstrate an increase in the density of acid sites with the reduction of the Si/Al ratio and a lower acidity strength than the zeolites.

Alumination can be done by the following process:

(A) directly by adding an aluminum precursor in the synthesis of the mesoporous material or indirectly by deposition of alumina on a mesoporous material by treating with at least one aluminum reagent, for example selected from

$AlCl_3$, $NaAlO_4$ Al $(NO_3)_3$, $Al(OR)_3$ where R is Chosen from linear or branched C1 - C6 alkyl groups, in order to obtain a compound whose Si/Al ratio is comprised between 0.1 and 1000.

(B) adding at least one catalytically active species selected from the group consisting of Group VIIIB and/or VIB metals preferably by ionic exchange or impregnation.

(C) drying, followed by heat and / or chemical treatment (reduction, sulfurization, etc.)

Optionally, step (B) may also comprise the addition of one or more doping metals selected from the group of rare earths or group IVB or IB and/or the addition of one or more other doping elements, for example chosen from chlorine, fluorine, boron or phosphorus. In particular, the addition of chlorine can increase the acidity of the material.

Preferably, the preferred metals of groups IVB and IB are Ti and Cu.

Generally, the steps of the above process are carried out in the order (A), (B), (C). However, it is possible to envisage a simultaneous implementation of steps (A) and (B), or even an implementation of step (B) before step (A).

In one embodiment of the present invention, deposition of alumina in step (A) is accomplished by grafting.

According to a preferred embodiment, the deposition of alumina in the silica is carried out by grafting according to the following steps:

(i) reacting the mesostructured silica with an aluminum-containing compound of the formula $Al(OR)_3$, wherein R is selected from linear or branched C1-C6 alkyl groups in the presence of an activating agent of the protons of the silanol groups of silica in a solvent whose water content is less than or equal to 0.005% by weight, preferably less than or equal to 0.0002%;

(ii) separating of the solid by filtration and then optionally washing the solid at least once with the same solvent as used in the previous step;

(iii) hydrolyzing of the Al(OR) groups grafted onto the silica by mixing the washed solid in a solution containing at least one alcohol of formula $R_1OH$, $R_1$ being chosen from linear or branched C1 - C6 alkyl groups and a stoichiometric amount of water.

(iv) Filtrating, washing of the solid obtained in an alcohol and drying

(v) Calcining of the washed and dried product.

Step (i) corresponds to the reaction (1):

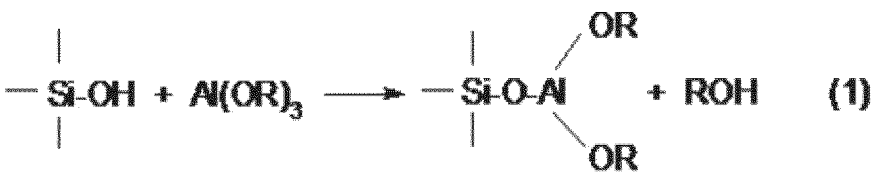

The stirring for step (i) is carried out for a period of 1 to 4 hours at a temperature of 20 to 95 °C, preferably 45 to 90 °C.

The solvent of step (i) can be chosen from hydrophobic solvents (i.e. apolar solvents), such as one or more selected from benzene, toluene, xylene, cyclohexane, n-hexane, pentane, isopropyl benzene, or any mixture thereof. With preference the solvent is toluene. Preferably the solvent is dehydrated by drying on a molecular sieve before use.

Advantageously, the deposition of alumina on the mesoporous solid will be carried out using aluminum tri-sec-butoxide as a source of aluminum and toluene containing triethylamine as solvent.

There are grafting methods in which aluminum tri-isopropoxide is used as the aluminum source [P. lengo, M. Di Serio, A. Sorrentino, V. Solinas, E. Santacesaria, Appl. Catal. A, 167 (1998) 85].

For step (i), the silanol group-activation agent of the silica will be chosen from organic basic compounds, for example amines, preferably triethylamine, nitriles, etc. The role of this agent consists in activating the protons of the surface silanol groups and thus accelerating the reaction (1). It is thus possible to reduce the reaction temperature, which may be 85 °C. Step (iii) corresponds to the reaction:

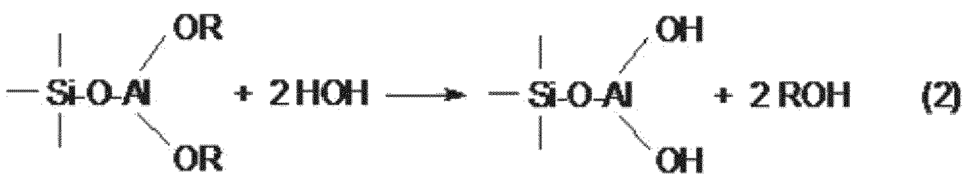

The hydrolysis step (iii) will preferably be carried out at ambient temperature for a period of from 0.1 to 48 hours, preferably from 1 to 36 hours. By "ambient temperature" is meant a temperature ranging from 18 to 25 °C, and in particular a temperature of 20 °C. The quantity of water required in step (iii) can for example be calculated by considering that $Al(OC_4H_9)_3$ completely adsorbs on the solid, taking into account an amount of stoichiometric water (duration less than 2 h). The drying of step (iv) can be carried out at a temperature of 80 to 130 °C. for 1 to 25 hours, optionally under air or nitrogen flow or even under vacuum. The calcination step (v) can be carried out at a temperature of 400 to 600 °C, preferably 400 to 550 °C, for a period of 0.5 to 8 hours, for example 1 to 6 hours, under a flow of gas. The step (A) for depositing alumina, for example by grafting according to steps (i) to (iv), can be repeated several times, generally from 2 to 10 times, in order to obtain a layer of alumina on the surface of the mesoporous solid.
In another embodiment, the grafting method consists in sodium aluminate. In a preferred embodiment, silica is suspended under stirring in an aqueous solution containing sodium aluminate (with a concentration calculated to have the required Si/Al ratio after graphting) for 15h, at room temperature. The sample which results in the sodium form (Na-Al-SBA-15) is filtered, washed with water, dried at 80°C, and calcined for 6h under air at 550°C.

In an embodiment, the mesoporous material is an Al-containing material, preferably Al modified silica type.

Advantageously, the mesoporous material of the catalyst of step b) is a mesoporous material of SBA-15 topology or is a mesoporous material of Al-modified silica type, preferably of a IV isotherm type. Preferably the catalyst is selected from Ni-Ai-SBA-15 or Ni-Al-$SiO_2$.

In an embodiment, the mesoporous material of the catalyst of step b) is a mesoporous material is COK-12.

In an embodiment, the catalyst consists of nickel deposited on a support being a mesoporous material, and the nickel content of the catalyst is at least 0.5 wt%, preferably at least 1.0 wt%, more preferably at least 1.5 wt%, even more preferably at least 2.0 wt% based to the total weight of the catalyst. The nickel content is determined according to UOP961-12.

In an embodiment, the catalyst consists of nickel deposited on a support being a mesoporous material, and the nickel content of the catalyst is at most 10.0 wt%, preferably at most 5.0 wt%, more preferably at most 4.0 wt%, even more preferably of at most 3.5 wt%, and most preferably of at most 3.0 wt% based to the total weight of the catalyst. The nickel content is determined according to UOP961-12.

In an embodiment, the catalyst shows large mesoporous pores having an average pore diameter of at least 2.0 nm, preferably of at least 5.0 nm, more preferably of at least 7.0 nm, and even more preferably of at least 7.5 nm. As known by the person skilled in the art, the size of the pore is determined by the surfactant used during the synthesis. A pore size of at least 7.0 nm can be obtained using $(EO)_{20}(PO)_{70}(EO)_{20}$ triblock copolymer (Pluronic P123, Aldrich) as surfactant during the synthesis of the catalyst. The pore size can be determined according to ASTM D 4641 - 94 (reapproved 2006).

In an embodiment, the catalyst shows mesoporous pores having a mesoporous pore volume of at least 0.1 mL/g as determined according to ASTM D 4641 - 94 (reapproved 2006), preferably at least 0.2 mL/g.

In an embodiment, the catalyst has a bulk Si/Al ratio ranging from 5 to 10, preferably from 6 to 8, as determined according to UOP961-12.

The catalyst has preferably a total surface area (i.e. BET surface area) in the range of about 100 $m^2/g$ to about 1000 $m^2/g$ prefereably from 120 $m^2/g$ to 700 $m^2/g$, more preferably from 150 $m^2/g$ to about 500 $m^2/g$ as determined by N2 sorption analysis according to ASTM D 4365 - 95 (reapproved 2008).

In an embodiment the catalyst has a sieve fraction ranging from 0.10 to 0.50 mm, preferably from 0.15 to 0.25 mm.

The above catalyst is useful for the conversion of ethylene from a diluted feedstream into olefins having at least four carbon atoms by oligomerization reaction.

The solids of the present invention can be used as itself as a catalyst. In another embodiment it can be formulated into a catalyst by combining with other materials that provide additional hardness or catalytic activity to the finished catalyst product. Materials which can be blended with can be various inert or catalytically active materials, or various binder materials. These materials include compositions such as kaolin and other clays, various forms of rare earth metals, phosphates, alumina or alumina sol, titania, zirconia, quartz, silica or silica sol, and mixtures thereof. These components are effective in densifying the catalyst and increasing the strength of the formulated catalyst. The catalyst may be formulated into pellets, spheres, extruded into other shapes, or formed into a spray-dried particles. The amount of mesoporous material which is contained in the final catalyst product ranges from 10 to 90 weight percent of the total catalyst, preferably 20 to 70 weight percent of the total catalyst.

The metals can be introduced either in the power form or to the extruded form.

**Test methods and definitions**

Total surface area was determined by N2 sorption analysis according to ASTM D 4365 - 95 (reapproved 2008).

Pore diameter and pore volume were determined according to D4641-94 (reapproved 2006).

Si/Al ratio and Ni content were determined according to UOP961-12.

Sieve fraction is determined in accordance to ASTM D4513 - 11.

Gas chromatography was performed on Columns: DB1 (40 m, 0.1 mm, 0.4 μm) and $Al_2O_3$ (50 m, 0.32 mm, 5 μm) using Agilent operated by ChemStation software.

## Examples

The advantages of the present invention are illustrated by the following examples. However, it is understood that the invention is by no means limited to the specific examples.

### Example 1: synthesis of a Ni-Al-SBA-15 catalyst

Preparation of the catalyst

The catalyst can be prepared according to the procedure described in "Heterogeneous oligomerization of ethylene over highly active and stable Ni-Al-SBA-15 mesoporous catalysts" by Andrei, R. D. et al, Journal of catalysis, 323 (2015) 76-84.

According to this procedure, pure siliceous SBA-15 was synthetized according to conventional method. Synthesis is performed in water using $(EO)_{20}(PO)_{70}(EO)_{20}$ triblock copolymer (Pluronic P123, Aldrich), Tetraethyl orthosilicate (TEOS, Aldrich) and HCl 2 M (Aldrich) with a molar ratio of 1 TEOS/0.016 P123/ 4.9 HCl/40.5 $H_2O$. The mixture is stirred for 24 hours at 40°C, and then it is maintained for 48 hours at 100°C in a Teflon line autoclave under static conditions. The solid product is filtered, washed with water and dried in an oven at 80°C overnight. The material is calcined in air flow at 550°C for 8 hours to obtain a white SBA-15 powder.

Al-containing material (Al-SBA-15) is obtained from SBA-15 silica by grafting with sodium aluminate. For this, 3.8 g of SBA-15 is suspended under stirring in 400 mL of aqueous solution containing 1.03 g of sodium aluminate for 15 h, at room temperature. The sample which results in sodium form (Na-Al-SBA-15) is filtered, washed with water, dried at 80°C in air and calcined for 6 hours in air at 550°C. 2g of Na-Al-SBA-15 is contacted three times, for 2 hours at 30°C, under constant agitation, with 100 $cm^3$ of 0.5 M aqueous solution of $NH_4NO_3$ to obtain the ammonium form, $NH_4$-Al-SBA-15. The sample in ammonium form is subjected to successive nickel-ion exchanges with a 0.5 M aqueous solution of nickel nitrate, following the same procedure as above. The exchanged sample is dried and then calcined for 5 hours at 550°C to obtain the catalyst Ni-Ai-SBA-15 of the invention.

Characterization of the catalyst is conducted on calcined samples

The Si/Al ratio in Al-SBA-15 samples is in the range 6-8. The amount of nickel in Ni-Al-SBA-15 is 2.6 wt% based on the total weight of the catalyst. This concentration corresponds to an exchange level of $NH_4^+$ ions with $Ni^{2+}$ of about 50%.The catalyst produced exhibits a type IV isotherm. The total surface area is 460 $m^2$/g. The pore diameter D is 7.9 nm.

### Example 2: Ethylene oligomerization

The ethylene oligomerization reaction was performed in a stainless fixed bed reactor using 2 g of catalyst. The pressure was regulated via a back pressure regulator. Thermocouple was placed on the top of the catalyst bed. Gas chromatography was connected on-line with the outlet of the reactor.

Prior the reaction, the catalyst Ni-Al-SBA-15, was activated in a reactor either in $N_2$ flows (5 NL/h), at 550°C for 8 hours, applying 60°C/h heating rate. After the activation the catalyst was cooled down to 150°C. The temperature was then increased gradiently up to a final reaction temperature of 200 or 250°C for the oligomerization reaction. For all tests, the reaction was conducted at a pressure of 1.5 MPa.

In the following tests, the feedstream comprised $C_2H_4$ and $N_2$ at a ratio 17-29 wt% ($C_2H_4$): 83-71 wt% ($N_2$) as based on the total weight of the feedstream. Conversion and selectivity was calculated based on the results of gas chromatography, making sure that all the peaks were integrated.

Regeneration of Ni-Ai-SBA-15 was carried out after every test. The catalyst was regenerated in a reactor in air flows (5 NL/h), at 550°C for 2 hours, applying 60°C/h heating rate and then in $N_2$ flows (5 NL/h) during 8 hours.

Conditions : Pressure P = 1.5 MPa, Temperature T = 250 °C, 17 wt% C2- in $N_2$ stream as based on the total weight of the feedstream, WHSV (feed) = 0.75 $h^{-1}$, TOS: 65 hours.

It can be seen that in accordance with the invention the ethylene partial pressure is about 0.3 MPa.

**Example 3: Ethylene oligomerization: Conversion and selectivity with Ni-Al-SBA-15 catalyst**

The conversion and selectivity with TOS are presented in Fig. 1. The catalyst is active, showing the conversion of more than 90% after a time of 40 hours on stream. The catalyst is stable for at least 70 hours. Product composition consists of about 90 wt% of butenes (C4) - octenes (C8)- fractions as based on the total weight of the products, about 5 wt% of ethane and about 5 wt% of C5+ paraffins including traces of uneven olefins. As it is seen in the Table 1, butenes-fraction is prevailling (about 60 wt%), where mainly internal olefins are formed.

Table 1: Product of ethylene oligomerization

| Product | Selectivity, wt% | Isomers | Selectivity, wt% |
|---|---|---|---|
| $C_{4-}$ | 61 | 1-$C_{4-}$ | 10 |
| | | 2-t-$C_{4-}$ | 31 |
| | | 2-c-$C_{4-}$ | 19 |
| | | i-$C_{4-}$ | 1 |
| $C_{6-}$ | 20 | 1-$C_{6-}$ | 0.5 |
| | | 2-$C_{6-}$ | 6.7 |
| | | i-$C_{6-}$ | 12.8 |
| $C_{8-}$ | 9 | $C_{8-}$ | 9 |
| $C_2$ | 5 | $C_2$ | 5 |
| $C_{3+}$ Paraffins, $C_{5-}$, $C_{7-}$ | 5 | $C_{3+}$ Paraffins, $C_{5-}$, $C_{7-}$ | 5 |

Conditions test: Pressure P = 1.5 MPa, Temperature T = 250 °C, 17 wt% C2- in $N_2$ stream as based on the total weight of the feedstream, WHSV (total feed) = 0.75 $h^{-1}$, TOS: 65 hours.

It can be seen that in accordance with the invention the ethylene partial pressure is about 0.3 MPa.

**Example 4: Ethylene oligomerization: Influence of the temperature with Ni-Al-SBA-15 catalyst**

Influence of the reaction temperature (200 and 250 °C) on the conversion, selectivity and stability has been tested. Other parameters such as WHSV, pressure and concentration of ethylene in the stream have been held constant. The results are presented in Fig. 2.

For the same TOS, the activity for two reaction temperatures can be considered the same (the difference is within the error margin). At 200 °C, the catalyst is more stable, showing lower deactivation rate, i.e, $2.9*10^{-4}$ vs. $1.6*10^{-3}$ $h^{-1}$ (250 °C). In terms of stability the same tendency remains for higher WHSV (feed) = 3.5 $h^{-1}$ (see Fig. 3).

**Example 5: Ethylene oligomerization: Influence of the WHSV with Ni-Al-SBA-15 catalyst**

The WHSV of the feed was increased from 1.75 up to 3.5 $h^{-1}$, which gives a WHSV of ethylene equal to 0.5 and 1 $h^{-1}$, respectively. The results are presented in Fig. 4.

Within the same reaction temperature (250 °C) and TOS (10h) deactivation rate decreased proportionally to the increase of WHSV (for WHSV (feed) 1.75 and 3.5 $h^{-1}$). For higher space velocity, the selectivity towards C4- olefins and towards total even olefins is increased. Surprisingly, it can be seen that selectivity toward olefins having 4 atoms of carbon is 70 wt% at a WHSV of 1.75 $h^{-1}$ and 65 wt% at a WHSV of 3.5 $h^{-1}$. Thus, the selectivity increases whereas the WHSV decreases.

The increase of selectivity toward C4- olefins in a C2 diluted stream when lowering the WHSV is surprising as the results obtained in "Heterogeneous oligomerization of ethylene over highly active and stable Ni-Al-SBA-15 mesoporous catalysts" by Andrei, R. D. et al, Journal of catalysis, 323 (2015) 76-84 showed the contrary, i.e. a decrease of the selectivity toward C4- olefins in correlation with the decrease WHSV as shown by the results of table 2 given as comparative example of the inventive process. Without being bound by a theory the inventors found that this might be an effect of the dilution of ethylene in the stream.

Table 2: Product of ethylene oligomerization (comparative)

| Pressure (MPa) | WHSV ($h^{-1}$) | Olefins wt% | | | |
|---|---|---|---|---|---|
| | | C4 | C6 | C8 | C10+ |
| 3 | 17.5 | 70 | 18 | 9 | 3 |
| 3 | 15.0 | 61 | 23 | 12 | 4 |
| 3 | 12.5 | 55 | 26 | 15 | 4 |
| 3 | 10.0 | 45 | 31 | 19 | 5 |

Conditions of comparative tests: Temperature T = 150 °C, 99 wt% $C_2$, TOS: about 1 hour, catalyst Ni-Al-SBA-15.

In the comparative test the ethylene partial pressure is about 3.0 MPa.

Fig. 5 shows space time yield for each case for the highest tested WHSV (3.5 $h^{-1}$). The isomers distribution within each olefin's fraction does not change much between the different conditions, i.e., mostly internal C4= olefins are formed.

**Example 6: synthesis of a Ni-Al-$SiO_2$ catalyst**

Preparation of the catalyst

The catalyst can be prepared according to the procedure described in "Nickel and molybdenum containing mesoporous catalysts for ethylene oligomerization and metathesis" by Andrei, R. D. et al, New J. Chem 40 (2016) 4146-4152.

According to this procedure, $SiO_2$ was synthetized according to the conventional method, using $(EO)_{20}(PO)_{70}(EO)_{20}$ triblock copolymer (Pluronic P123, Aldrich), Tetraethyl orthosilicate (TEOS, Aldrich) and HCl 2 M (Aldrich) with a molar ratio of 1 TEOS/0.016 P123/ 4.9 HCl/40.5 $H_2O$. The mixture is stirred for 24 hours at 40°C, and then it is maintained for 48 hours at 100°C in a Teflon-lined autoclave under static conditions. The solid product is filtered, washed with water and dried in an oven at 80°C overnight. The material is calcined in air flow at 550°C for 8 hours.

Al-Si-$O_2$ is obtained from $SiO_2$ by grafting with sodium aluminate (54+/- 1 % $Al_2O_3$ Carlo Erba). For this, 4.0 g of silica were suspended under stirring in 400 mL of aqueous solution containing 1.1 g of sodium aluminate for 15 h, at 25°C, corresponding to a Si/Al ratio of 5. The sample which results in sodium form (Na-Al-$SiO_2$) was subjected to successive ion exchange with $NH_4NO_3$ (99+%, Acros Organics) and $Ni(NO_3)_2 \cdot 6H_2O$ (98%, Alfa Aesar). Typically 2 g of Na-Al-$SiO_2$ were contacted three times, for 2 h at 25°C, under constant agitation, with 100 $cm^3$ of the 0.5 M aqueous solution of $NH_4NO_3$ to obtain the ammonium form $NH_4$-Al-$SiO_2$. The sample in ammonium form was subjected to successive nickel-ion exchanges with a 0.5 M aqueous solution of nickel nitrate, following the same procedure as above. The exchanged sample is dried and then calcined for 5 hours at 550°C to obtain the catalyst Ni-Al-$SiO_2$ of the invention.

The $SiO_2$ used in the catalyst had a pore diameter of 10-12 nm, and total surface area was ranging from 160-250 $m^2$/g.

**Example 7: Ethylene oligomerization: Conversion and selectivity with Ni-Al-$SiO_2$ catalyst**

The conversion and selectivity with a time on stream of 75 hours are presented in Fig. 6. The conversion of ethylene was more 80 % after a time of 40 hours on stream for both fresh and regenerated catalyst. The catalyst is stable for at least 75 hours. Product composition is shown on figure 6. As it can be see C4- fraction is prevail (about 75 wt% as based on the total weight of the product composition).

The tests were conducted in a flow mode using a fixed-bed reactor with a time on stream (TOS) of 75 hours, at a temperature of 250°C, under a pressure of 1.5 MPa with a feed of 1 L/h of $N_2$ and 0.2 L/h of ethylene (corresponding to 17 wt% C2 in $N_2$ stream as based on the total weight of the feedstream and to a WHSV (feed) of 1.1 $h^{-1}$).

This can be compared to the results obtained in "Nickel and molybdenum containing mesoporous catalysts for ethylene oligomerization and metathesis" by Andrei, R. D. et al, New J. Chem 40 (2016) 4146-4152; wherein the catalytic behavior of the of the Ni-Al-$SiO_2$ catalyst was evaluated for ethylene oligomerization carried out in the flow mode, using a fixed-bed reactor. Initial conversion of ethylene was about 90 wt% and it declined smoothly to reach about 80 wt% after 9 hours (see figure 5 of the document). The product distribution did not change during the catalytic test. The results are given in table 3 as comparative example of the inventive process.

Table 3: Product of ethylene oligomerization (comparative)

| Product | Selectivity, wt% | Isomers | Selectivity, wt% |
|---|---|---|---|
| $C_{4-}$ | 47 | 1-$C_{4-}$ | 18 |
| | | 2-t-$C_{4-}$ | 38 |
| | | 2-C-$C_{4-}$ | 44 |
| $C_{6-}$ | 34 | - | |
| $C_{8-}$ | 16 | | |
| $C_{10+}$ | 3 | | |

Conditions comparative test: Pressure P = 3.0 MPa, Temperature T = 250 °C, 99 wt% C2 as based on the total weight of the feedstream, WHSV (feed) = 10 $h^{-1}$, Time on stream: 9 hours.

Comparison of inventive and comparative examples show an increase of the selectivity toward C4- products according to the inventive process.

## Claims

1. Process for the conversion of ethylene into olefins having at least four carbon atoms **characterized in that** the process comprises:

   a) providing a feedstream comprising from 0.5 to 50 wt% of ethylene as based on the total weight of the feedstream;
   b) putting the feedstream in contact with a catalyst at a temperature ranging from 80 to 500°C, under a pressure ranging from 0.5 to 2.9 MPa; and at a weight hourly space velocity (WHSV) of the feedstream ranging from 0.1 to 10.0 $h^{-1}$;
   c) recovering the olefins having at least four carbon atoms;

   and **in that** the catalyst of step b) consists of one or more metals of group VIIIB and/or of group VIB, deposited on a support being a mesoporous material.

2. The process according to claim 1 **characterized in that** the mesoporous material of the catalyst of step b) is an ordered mesoporous material or any type of mesoporous material based on silica.

3. The process according to any one of claims 1 to 2 **characterized in that** the one or more metals of group VIIIB and/or of group VIB, of the catalyst of step b) are selected from nickel, cobalt, chromium, molybdenum, tungsten, palladium and any mixture thereof.

4. The process according to any one of claims 1 to 3 **characterized in that** the catalyst of step b) consists of nickel deposited on a support being a mesoporous material, and preferably **in that** the catalyst comprises from 0.5 to 10.0 wt% of nickel based on the total weight of the catalyst as determined according to UOP961-12.

5. The process according to any one of claims 1 to 4, **characterized in that** the catalyst of step b) is selected from Ni-Ai-SBA-15 or Ni-Al-$SiO_2$.

6. The process according to any one of claims 1 to 5 **characterized in that**:

   - the catalyst of step b) has a total surface area ranging from 100 to 1000 $m^2/g$, preferably from 120 to 700 $m^2/g$ as determined by $N_2$ sorption analysis according to ASTM D 4365 - 95 (reapproved 2008); and/or
   - the catalyst of step b) has a bulk Si/Al ratio ranging from 5 to 10 as determined according to UOP961-12.

7. The process according to any one of claims 1 to 6 **characterized in that** the catalyst of step b) comprises mesoporous pores having an average diameter of at least 2 nm as determined according to ASTM D 4641 - 94 (reapproved 2006), preferably the catalyst of step b) comprises mesoporous pores having an average diameter of at least 2 nm as determined according to ASTM D 4641 - 94 (reapproved 2006) and a mesoporous pore volume of at least 0.1

mL/g as determined according to ASTM D 4641 - 94 (reapproved 2006).

**8.** The process according to any one of claims 1 to 7 **characterized in that** the feedstream is put into contact with the catalyst at a temperature ranging from 100°C to 400°C, preferably from 150 to 350°C, more preferably at a temperature ranging from 180 to 280°C.

**9.** The process according to any one of claims 1 to 8 **characterized in that** the feedstream is put into contact with the catalyst at a pressure ranging from 0.7 to 2.0 MPa, and most preferably from 1.0 to 1.5 MPa.

**10.** The process according to any one of claims 1 to 9 **characterized in that** the feedstream is put into contact with the catalyst at a weight hourly space velocity (WHSV) of the feedstream of from 0.5 to 8.0 $h^{-1}$, preferably from 1.0 to 5.0 $h^{-1}$.

**11.** The process according to any one of claims 1 to 10 **characterized in that** the feedstream comprises from 10 to 30 wt% of ethylene based on the total weight of the feedstream.

**12.** The process according to any one of claims 1 to 11 **characterized in that** the process is carried out without replacement or reactivation of the catalyst during more than 40 hours, preferably more than 50 hours, more preferably more than 60 hours, and even more preferably more than 70 hours.

**13.** The process according to any one of claims 1 to 12 **characterized in that** the feedstream comprises less than 90 wt ppm of CO and less than 1.0 wt% of hydrogen and preferably less than 5%wt more preferably less than 2%wt of $H_2O$ as based on the total weight of the feedstream.

**14.** Use of a catalyst consisting of one or more metals of group VIIIB and/or of group VIB deposited on a support being a mesoporous material, in a process to convert ethylene into olefins having at least four carbon atoms according to any one of claims 1 to 13, **characterized in that** the process comprises a step wherein the feedstream is put in contact with said catalyst at a temperature ranging from 80 to 500°C, under a pressure ranging from 0.5 to 2.9 MPa and at a weight hourly space velocity (WHSV) of the feedstream ranging from 0.1 to 10.0 $h^{-1}$; and **in that** the feedstream comprises from 0.5 to 50 wt% of ethylene as based on total weight of the feedstream, preferably the catalyst is nickel deposited on a mesoporous material of SBA-15 topology or nickel deposited on a mesoporous material of Al-modified silica type.

**15.** The use of claim 14 **characterized in that** the feedstream comprising ethylene is an effluent of a process selected from Methanol To Olefin process (MTO), ethane/naphtha cracker process or oxidative coupling of methane process (OCM), or is or is a fluid catalytic cracking (FCC) off-gases.

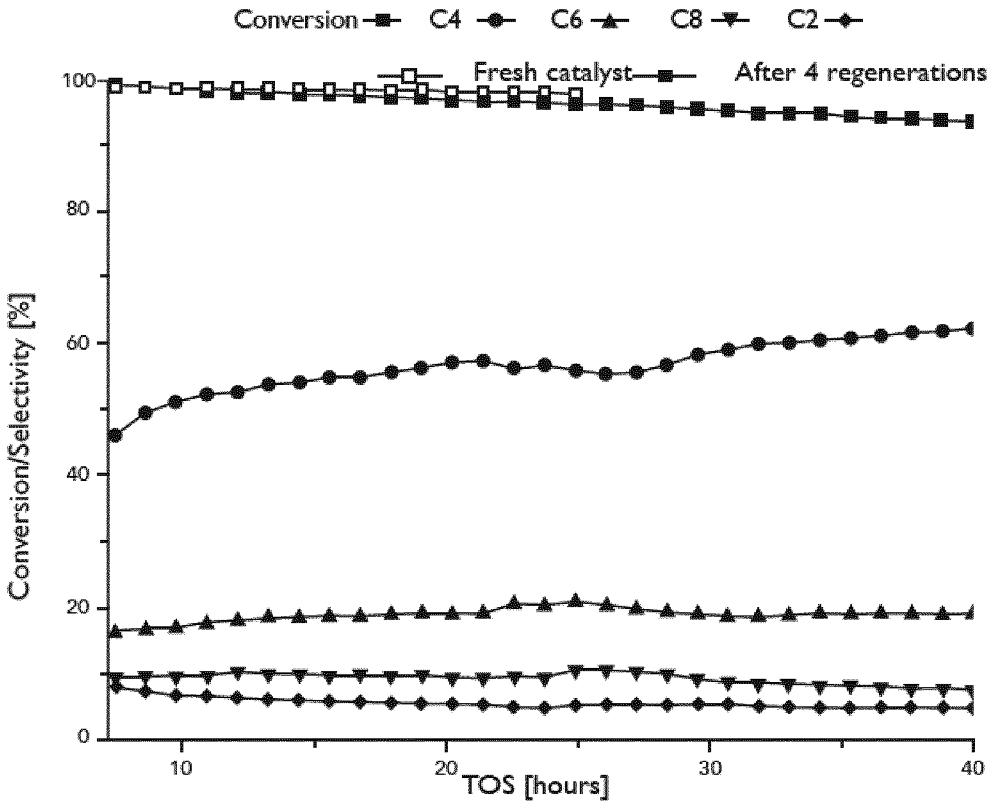


Figure 1

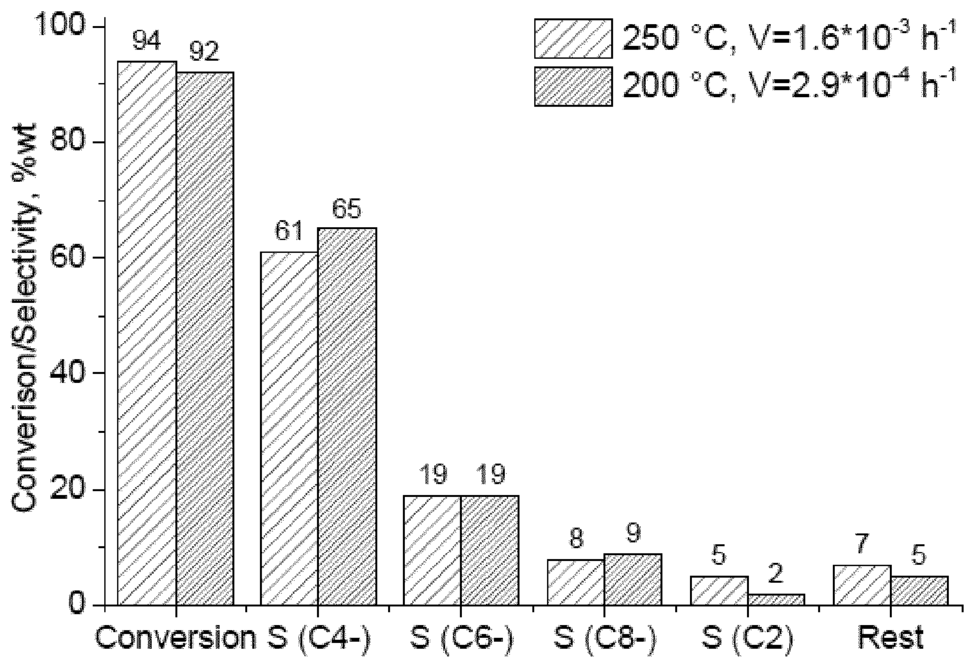


Figure 2

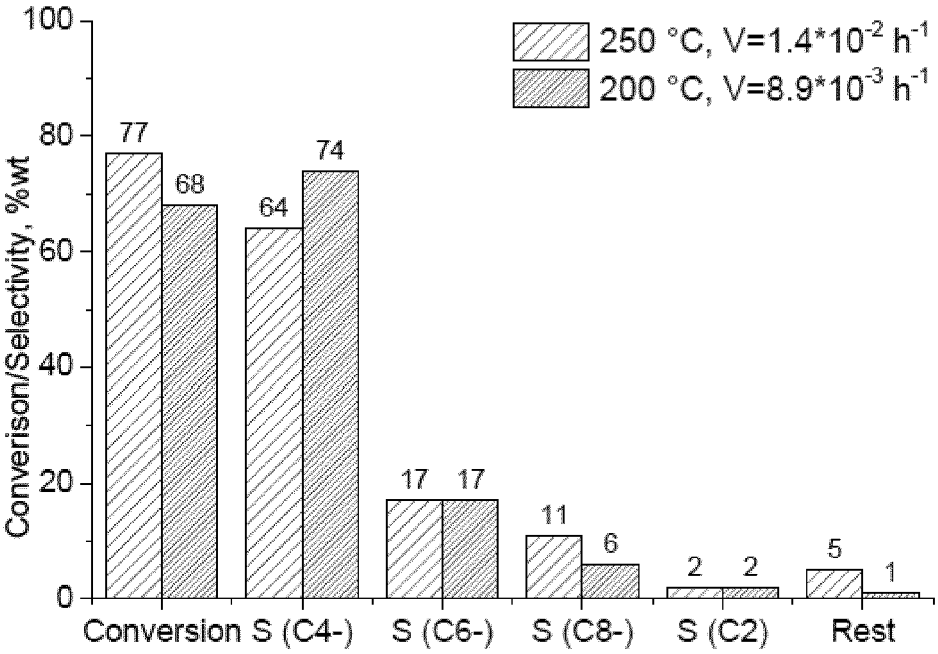


Figure 3

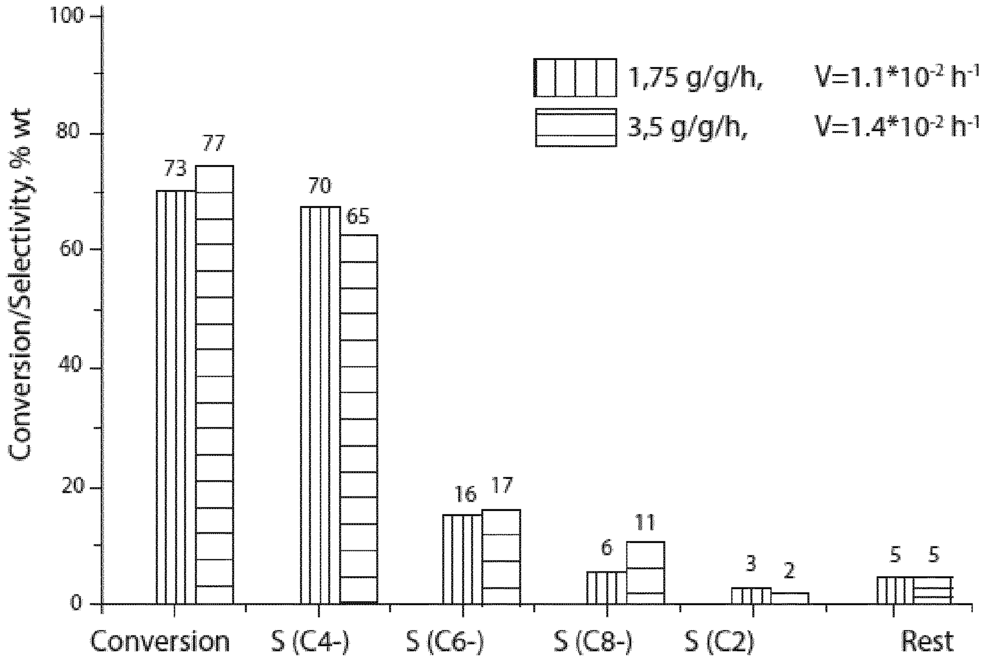


Figure 4

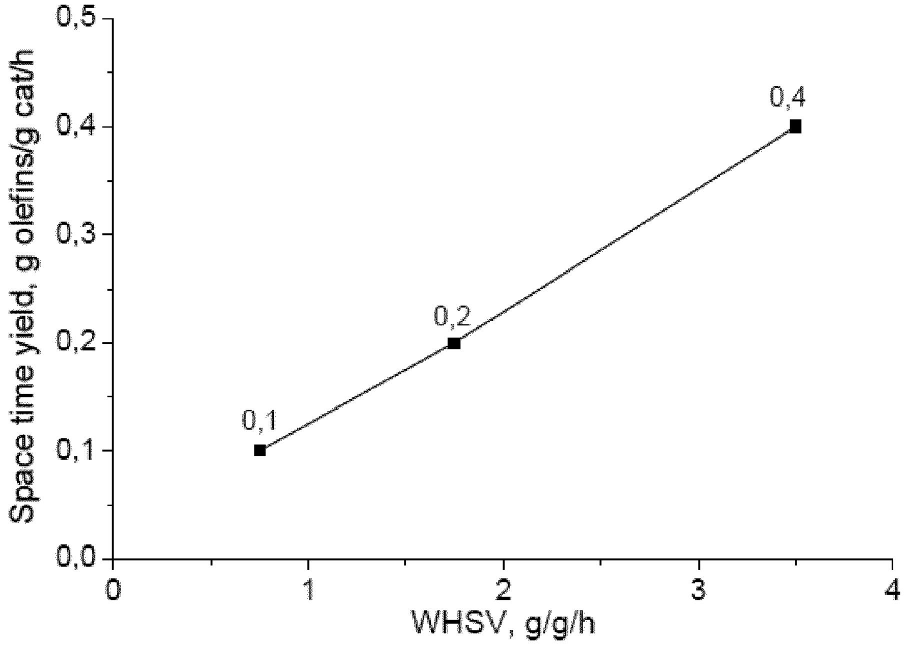


Figure 5

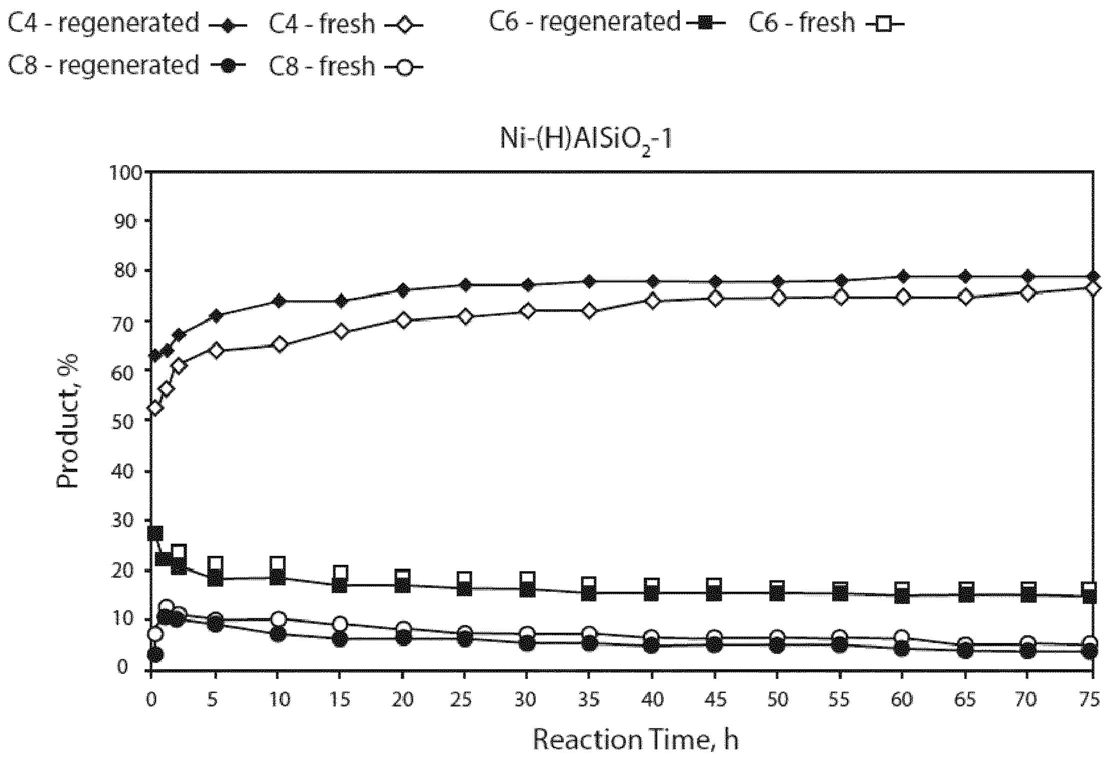


Figure 6

Europäisches Patentamt
European Patent Office
Office européen des brevets

# EUROPEAN SEARCH REPORT

Application Number
EP 17 30 5565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/275669 A1 (DAAGE MICHEL [US] ET AL) 18 September 2014 (2014-09-18)<br>* paragraph [0023]; claims; figures *<br>* paragraph [0027] - paragraph [0029] *<br>----- | 1-15 | INV.<br>C07C2/12 |
| X | US 5 449 849 A (DIGUISEPPI FRANK T [US] ET AL) 12 September 1995 (1995-09-12)<br>* abstract; claims; examples; tables *<br>----- | 1-15 | |

TECHNICAL FIELDS SEARCHED (IPC)
C07C

The present search report has been drawn up for all claims

2

| Place of search | Date of completion of the search | Examiner |
|---|---|---|
| The Hague | 4 January 2018 | van Laren, Martijn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 30 5565

This annex lists the patent family members relating to the patent documents cited in the above-mentioned European search report. The members are as contained in the European Patent Office EDP file on
The European Patent Office is in no way liable for these particulars which are merely given for the purpose of information.

04-01-2018

| Patent document cited in search report | Publication date | Patent family member(s) | Publication date |
|---|---|---|---|
| US 2014275669 A1 | 18-09-2014 | CA 2897970 A1 | 25-09-2014 |
| | | EP 2970043 A1 | 20-01-2016 |
| | | SG 10201707447Q A | 30-10-2017 |
| | | SG 11201505682X A | 28-08-2015 |
| | | US 2014275669 A1 | 18-09-2014 |
| | | WO 2014149731 A1 | 25-09-2014 |
| US 5449849 A | 12-09-1995 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2257366 A
- US 7279138 B

### Non-patent literature cited in the description


- **ANDREI, R. D. et al.** Heterogeneous oligomerization of ethylene over highly active and stable Ni-Al-SBA-15 mesoporous catalysts. *Journal of Catalysis,* 2015, vol. 323, 76-84
- **ANDREI, R. D. et al.** Nickel and molybdenum containing mesoporous catalysts for ethylene oligomerization and metathesis. *New J. Chem,* 2016, vol. 40, 4146-4152
- **C.T. KRESGE ; M. E. LEONOWICZ ; W.J. ROTH ; J.C. VARTULI ; J.S. BECK.** *Nature,* 1992, vol. 359, 710
- **A. CORMA ; V. FORNES ; T. NAVARRO ; J. PEREZ-PARIENTE.** *Journal of Catalysis,* 1994, vol. 148, 569
- **MOKAYA.** *Journal of Catalysis,* 2000, vol. 193, 103
- **S. C. SHEN ; S. KAWI.** *Chemistry Letters,* 1999, vol. 28, 1293
- **R. MOKAYA ; W. JONES.** *Chemical Communications,* 1997, 2185
- **Z. LUAN ; M. HARTMANN ; D. ZHAO ; W. ZHOU ; L. KEVAN.** *Chemistry of materials,* 1999, vol. 11, 1621
- **A. JENTYS ; N.H. PHAM ; H. VINEK.** *Journal of the Chemical Society, Faraday Transaction,* 1996, vol. 62, 3287
- **P. LENGO ; M. DI SERIO ; A. SORRENTINO ; V. SOLINAS ; E. SANTACESARIA.** *Appl. Catal. A,* 1998, vol. 167, 85
- **ANDREI, R. D. et al.** Heterogeneous oligomerization of ethylene over highly active and stable Ni-Al-SBA-15 mesoporous catalysts. *Journal of catalysis,* 2015, vol. 323, 76-84